# EUROPEAN PATENT APPLICATION

(11) **EP 4 768 895 A1**
(43) Date of publication of application: **01.07.2026**
(21) Application number: 24856511.1
(22) Date of filing: 22.08.2024
(51) Int. Cl.: G01N 21/27, A61B 5/1455, G01N 21/359

(54) **ANALYSIS METHOD AND DETERMINATION SYSTEM**

(30) Priority: 22.08.2023 JP 2023134494
(71) Applicant: The Kitasato Institute, Tokyo 108-8641 (JP); TOKYO UNIVERSITY OF SCIENCE FOUNDATION, Tokyo 162-8601 (JP)
(72) Inventor: NABENISHI Hisashi, Tokyo 108-8641 (JP); AIKAWA Naoyuki, Tokyo 162-8601 (JP)
(74) Representative: Bryn Aarflot AS
(86) International application number: PCT/JP2024/029801
(87) International publication number: WO 2025/041823

(57) **Abstract**

An analysis method comprising: a first step of obtaining feature values relating to a plurality of values corresponding to wavelengths from image data of a blood vessel of a target animal, the image data being generated by capturing an image of a shallow part of a body surface (vein) of the animal with a multispectral camera; and a second step of estimating a concentration of a blood component contained in blood in the blood vessel based on the feature values.

## Description

### TECHNICAL FIELD

The present invention relates to an analysis method and a determination system.

Priority is claimed on Japanese Patent Application No. 2023-134494, filed August 22, 2023, the contents of which are incorporated herein by reference.

### BACKGROUND ART

Blood testing of cattle is referred to as a metabolic profile test. The metabolic profile test is a nutritional monitoring method for cattle herds, primarily based on blood biochemical analysis. Conducting metabolic profile tests is expected to improve milk yield and milk components, prevent perinatal diseases such as ketosis and milk fever, and enhance reproductive performance. Metabolic activities evaluated by the metabolic profile test include, for example, energy metabolism, protein metabolism, lipid metabolism, liver disorders, minerals, and body condition score (BCS).

As a specific method for the metabolic profile test, for example, a method is known in which components related to the aforementioned metabolic activities, contained in blood collected from cattle, are analyzed using a clinical chemistry analyzer (see, for example, Non-Patent Literature 1).

### Citation List

### Non Patent Literature

Non-Patent Literature 1: Takayuki Watanabe, "Metabolic Profile Test," Chikusan Gijutsu (Animal Husbandry Technology), February 2020 issue.

### SUMMARY OF INVENTION

### Technical Problem

However, blood collection for sampling not only places a significant burden on the cattle but is also restricted to being performed by veterinarians. Therefore, it is difficult to promote the widespread use of the metabolic profile test.

Furthermore, the metabolic profile test requires an expensive clinical chemistry analyzer to analyze the blood. Therefore, blood analysis must typically be outsourced to medical or research institutions. Consequently, it takes a considerable amount of time to obtain the test results.

As described above, the metabolic profile test has the following problems: not only is the cattle's body harmed during blood collection, but it is also impossible to ascertain the health condition of the cattle on the spot, leading to a risk that the health condition of the cattle changes while the analysis is being performed.

The present invention has been made in view of the above circumstances, and an object thereof is to provide an analysis method and a determination system capable of non-invasively ascertaining the health condition of a target animal on the spot.

### Solution to Problem

The embodiments of the present invention are as follows.
[1] An analysis method comprising:
   a first step of obtaining feature values relating to a plurality of values corresponding to wavelengths from image data of a blood vessel of a target animal, the image data being generated by capturing an image of a shallow part of a body surface (vein) of the animal with a multispectral camera; and
   a second step of estimating a concentration of a blood component contained in blood in the blood vessel based on the feature values.
[2] The analysis method according to [1], wherein the plurality of values corresponding to wavelengths are values relating to absorbance of the blood in the blood vessel.
[3] The analysis method according to [1] or [2], wherein:
   in the first step, at least n (where n ≥ 228) values are obtained at wavelengths of 900 nm or more and 1,694 nm or less; and
   in the second step, the concentration of the blood component is classified into classes according to the concentration of the blood component, based on feature values relating to a plurality of values including the n values.
[4] The analysis method according to any one of [1] to [3], wherein the image data of the blood vessel is image data of a ventral side of a tail (median caudal vein) of the target animal.
[5] A determination system comprising:
   an imaging device that captures an image of a body surface of a target animal;
   a learning device that obtains feature values relating to a plurality of values corresponding to wavelengths from image data of a blood vessel of the animal generated by the imaging device, and learns the feature values; and
   a determination device that estimates a concentration of a blood component contained in blood in the blood vessel based on the feature values.

### Advantageous Effects of Invention

The present invention can provide an analysis method and a determination system capable of non-invasively ascertaining the health condition of a target animal on the spot.

### BRIEF DESCRIPTION OF DRAWINGS

FIG. 1 is a diagram showing a flow of an analysis method according to an embodiment of the present invention.
FIG. 2 is a diagram illustrating an example of classifying concentrations of a blood component into classes according to the concentrations of the blood component in the analysis method according to an embodiment of the present invention.
FIG. 3 is a diagram illustrating an example of classifying concentrations of a blood component into classes according to the concentrations of the blood component in the analysis method according to an embodiment of the present invention.
FIG. 4 is a diagram illustrating an example of classifying concentrations of a blood component into classes according to the concentrations of the blood component in the analysis method according to an embodiment of the present invention.
FIG. 5 is a schematic block diagram showing a determination system according to an embodiment of the present invention.
FIG. 6 is a schematic block diagram showing a specific example of a functional configuration of a learning device of the determination system according to an embodiment of the present invention.
FIG. 7 is a flowchart showing a specific example of processing of the learning device of the determination system according to an embodiment of the present invention.
FIG. 8 is a schematic block diagram showing a specific example of a functional configuration of a determination device of the determination system according to an embodiment of the present invention.
FIG. 9 is a flowchart showing a specific example of processing of the determination device of the determination system according to an embodiment of the present invention.
FIG. 10 is a diagram schematically showing a hardware configuration example of an information processing apparatus 90 applied to the present invention.
FIG. 11 is a diagram showing an absorption spectrum obtained in Example 1 by obtaining 46 values relating to absorbance in blood of cattle corresponding to wavelengths of 900 nm or more and 1,694 nm or less from image data of a blood vessel of cattle generated by a multispectral camera.
FIG. 12 is a diagram showing an overview of training and validation of a classification model in Example 2.

### DESCRIPTION OF EMBODIMENTS

Embodiments for providing the analysis method and the determination system of the present invention will be described.

Further, these embodiments are intended to provide specific explanations to make the purport of the invention more readily understandable, and are not intended to limit the present invention unless specifically indicated otherwise.

### [Analysis Method]

The analysis method according to an embodiment of the present invention includes: a first step of obtaining feature values relating to a plurality of values corresponding to wavelengths from image data of a blood vessel of a target animal, the image data being generated by capturing an image of a shallow part of a body surface (vein) of the target animal with a multispectral camera; and a second step of estimating a concentration of a blood component contained in blood in the blood vessel based on the feature values. The multispectral camera used in the present embodiment is an apparatus that captures images with a spectral resolution higher than at least that of a camera that performs RGB imaging. A hyperspectral camera may be used as the multispectral camera.

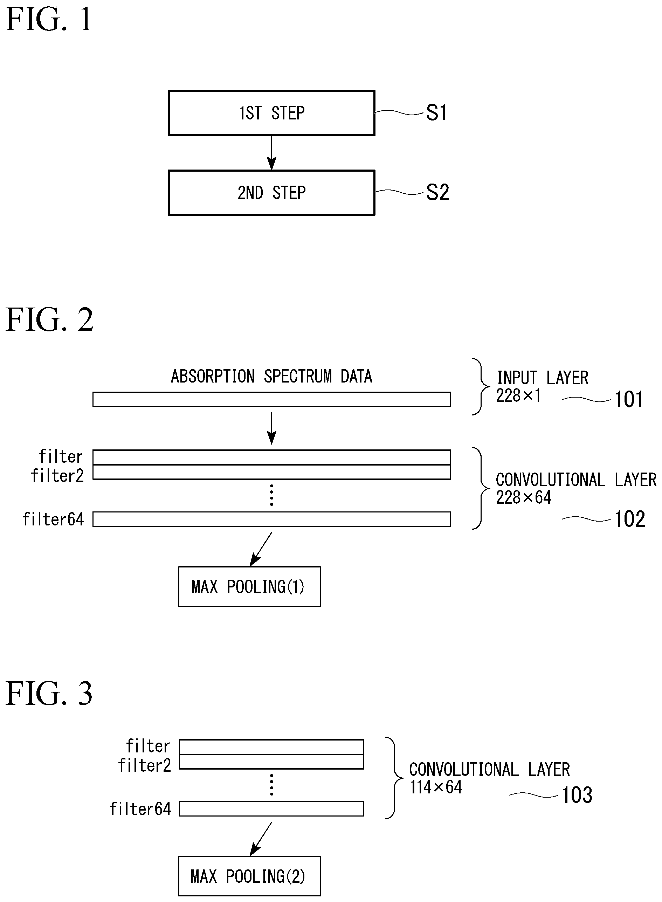

FIG. 1 is a diagram showing a flow of an analysis method according to an embodiment of the present invention.

### [First Step]

In the first step, first, a shallow part of a body surface (vein) of a target animal is captured with a multispectral camera to generate image data of a blood vessel of the animal (S1).

The target animal to be evaluated by the analysis method of the present embodiment is not particularly limited, and examples thereof include livestock such as cattle, pigs, horses, dogs, and chickens, as well as other animals raised by humans in ranches, zoos, and the like.

The part of the target animal to be captured by the multispectral camera is not particularly limited, but is preferably a part where no hair grows and blood vessels can be visually confirmed from the body surface, such as the inside of an animal's ear or the base of an animal's tail. When capturing an image of a part where hair grows, the hair in that part is shaved so that blood vessels can be visually confirmed from the body surface. In a case where the target animal is cattle, the base of the tail is preferable as the aforementioned part because no hair grows at the base of the tail of cattle. The part may be the ventral side of the base of the tail.

A multispectral camera is a spectral camera that performs imaging at a plurality of wavelengths. Image data captured by the multispectral camera includes information for each wavelength in addition to two-dimensional planar data in the x and y directions.

In the first step, feature values relating to a plurality of values corresponding to wavelengths are obtained from the image data of the blood vessel of the animal generated by the multispectral camera. Examples of the plurality of values corresponding to wavelengths include values relating to the absorbance (absorption spectrum) of the blood in the blood vessel. Among these, the values relating to the absorbance of the blood in the blood vessel are preferable because different absorption spectra are exhibited for the respective components contained in the blood in the blood vessel.

In the first step, it is preferable to obtain at least n (where n ≥ 228) values corresponding to wavelengths at wavelengths of 900 nm or more and 1,694 nm or less.

Examples of components contained in the blood in the blood vessel, for which feature values relating to a plurality of values corresponding to wavelengths can be obtained from image data generated by a multispectral camera, include glucose (evaluation of energy metabolism), blood urea nitrogen (evaluation of protein metabolism), total cholesterol (evaluation of lipid metabolism), calcium (diagnosis of minerals), Vitamin A, and the like. These components may be analyzed alone, or two or more thereof may be analyzed in combination. Since the feature values (for example, absorption spectra) of the respective components exhibit unique characteristics, the feature values relating to the components can be extracted even when a plurality of components are simultaneously analyzed.

### [Second Step]

In the second step, the concentration of a blood component contained in blood in the blood vessel is estimated based on the feature values (for example, absorption spectra) obtained in the first step (S2).

To estimate the concentration of the blood component, a database is created in advance regarding feature values related to a plurality of values corresponding to wavelengths. The feature values are obtained from actual measured values of the components and image data generated by the multispectral camera, with respect to blood collected from a blood vessel in advance. The concentration of the blood component contained in the blood in the blood vessel is estimated by comparing the database with the feature values related to a plurality of values corresponding to wavelengths obtained from the image data of the blood vessel of the animal.

Alternatively, to estimate the concentration of the blood component, feature values relating to a plurality of values corresponding to wavelengths are obtained from the actual measured values of the component and the image data of the blood vessel of the animal, and a database relating to the feature values is created. The concentration of the blood component contained in the blood in the blood vessel is estimated by comparing the database with the feature values related to a plurality of values corresponding to wavelengths obtained from the image data of the blood vessel of the animal.

Since an absorption spectrum is susceptible to the influence of light interference, it is preferable to use a five-point moving average method as a method for estimating the concentration of the blood component in order to remove noise. In the analysis method of the present embodiment, the five-point moving average method sets five arbitrary points in a wavelength range for measuring the absorbance of the blood in the blood vessel, and averages the absorbances in those wavelength ranges.

In the second step, it is preferable that the concentration of the blood component is classified into classes according to the concentration of the blood component, based on feature values relating to a plurality of values including the n values obtained in the first step.

Here, an example of classifying the concentration of a blood component into classes according to the concentration of the blood component will be described with reference to FIGs. 2 to 4.

As shown in FIG. 2, in order to remove noise included in the data of the absorption spectrum obtained in the first step, moving average processing of the absorption spectrum is performed in an input layer 101.

Subsequently, various feature values (specific frequency components) included in the absorption spectrum are extracted in a convolutional layer 102 using 64 types of filters. The convolutional layer 102 has 64 types of filters for 228 pieces of spectrum data. In the convolutional layer 102, an operation is performed, which multiplies an m × 1 (m is a positive integer) filter by 228 × 1 spectrum data and adds them while shifting the filter horizontally. For example, an operation of multiplying an 8 × 1 filter by 228 × 1 spectrum data and adding them while shifting the filter horizontally is performed. The obtained feature values (specific frequency components) are subjected to max pooling processing (1) to reduce the number of data by half. In the max pooling processing (1), a representative value of a certain region of a feature map is extracted. A maximum value in a 2 × 1 feature map is determined, and the number of data is reduced by half while sliding the feature map.

Next, as shown in FIG. 3, for the data that has been reduced by half by the max pooling processing (1), feature values are extracted again in a convolutional layer 103 using 64 types of filters. In the convolutional layer 103, "114" indicates that the spectrum data has been reduced to half of 228 by the max pooling processing (1). In addition, in the convolutional layer 103, "64" indicates that there are 64 types of filters, similar to the convolutional layer 102. In the convolutional layer 103, an operation is performed, which multiplies an m × 1 (m is a positive integer) filter by 114 × 1 spectrum data and adds them while shifting the filter horizontally. For example, an operation of multiplying an 8 × 1 filter by 114 × 1 spectrum data and adding them while shifting the filter horizontally is performed.

Subsequently, the obtained feature values (specific frequency components) are subjected to max pooling processing (2) to further reduce the number of data by half. In the max pooling processing (2), a representative value of a certain region of a feature map is extracted. A maximum value in a 2 × 1 feature map is determined, and the number of data is reduced by half while sliding the feature map.

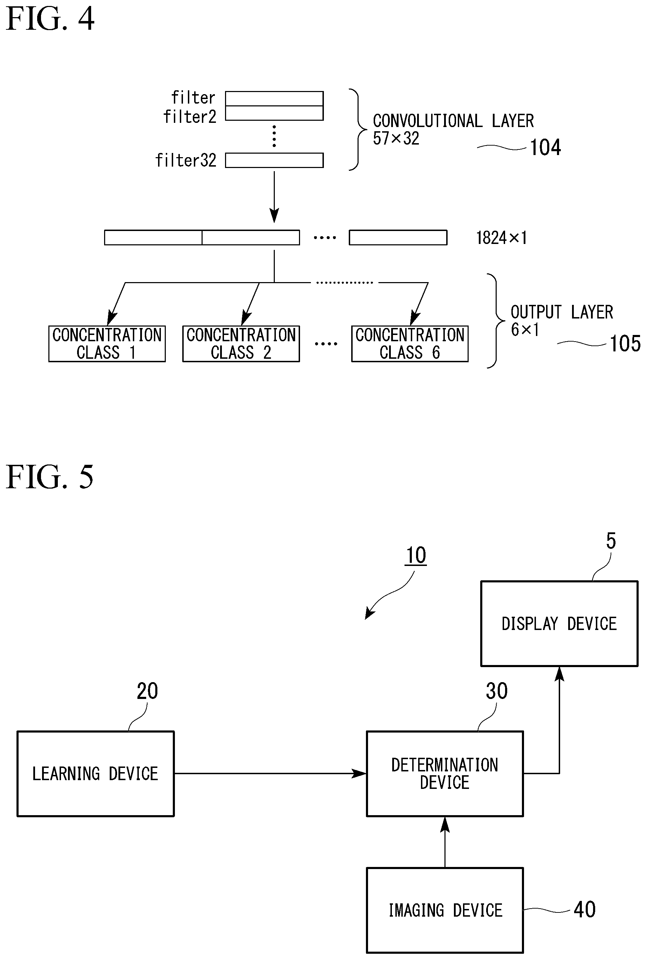

Finally, as shown in FIG. 4, various feature values (specific frequency components) included in the absorption spectrum are extracted in a convolutional layer 104 using 32 types of filters. In the convolutional layer 104, "57 × 32" indicates that there are 32 types of filters and 57 spectrum data, which is half of the 114 spectrum data of the convolutional layer 103. In FIG. 4, "1824 × 1" is obtained by arranging the results of 57 × 32 horizontally.

Then, an output layer 105 is a result of dividing the blood component concentration into six classes.

According to the analysis method of the present embodiment, since the first step and the second step are included, the health condition of a target animal can be grasped non-invasively on the spot.

According to the analysis method of the present embodiment, by capturing an image of a shallow part of a body surface (vein) of the target animal with a multispectral camera, a thick median caudal vein located in a shallow subcutaneous part can be directly imaged.

According to the analysis method of the present embodiment, in the second step, the concentration of the blood component is classified into classes according to the concentration of the blood component, so that it can be determined whether or not a component value deviates from a reference value in the target animal.

According to the analysis method of the present embodiment, since the image data of the blood vessel is image data of a ventral side of a tail (median caudal vein) of the target animal, the thick median caudal vein located in the shallow subcutaneous part can be directly imaged.

### [Determination system]

FIG. 5 is a schematic block diagram showing a determination system according to one embodiment of the present invention.

The determination system 10 of the present embodiment includes a learning device 20, a determination device 30, and a display device 5. An imaging device 40 that captures a multispectral image is connected to the determination device 30. The imaging device 40 captures an image with spectral resolution higher than at least that of a camera that performs RGB imaging. The imaging device 40 is, for example, a multispectral camera or a hyperspectral camera. The imaging device 40 is a camera that captures an image of a body surface of a target animal. Examples of the imaging device 40 include a near-infrared spectrometer "S-G1" manufactured by nanoseed Inc., and a multi/hyperspectral camera "XIMEA-SNAPSHOT" manufactured by XIMEA.

The learning device 20 and the determination device 30 may be communicatively connected via a network. The network may be a network using wireless communication, or may be a network using wired communication. The network may be configured using the Internet, for example, or may be configured using a local area network (LAN). The network may be configured by combining a plurality of networks.

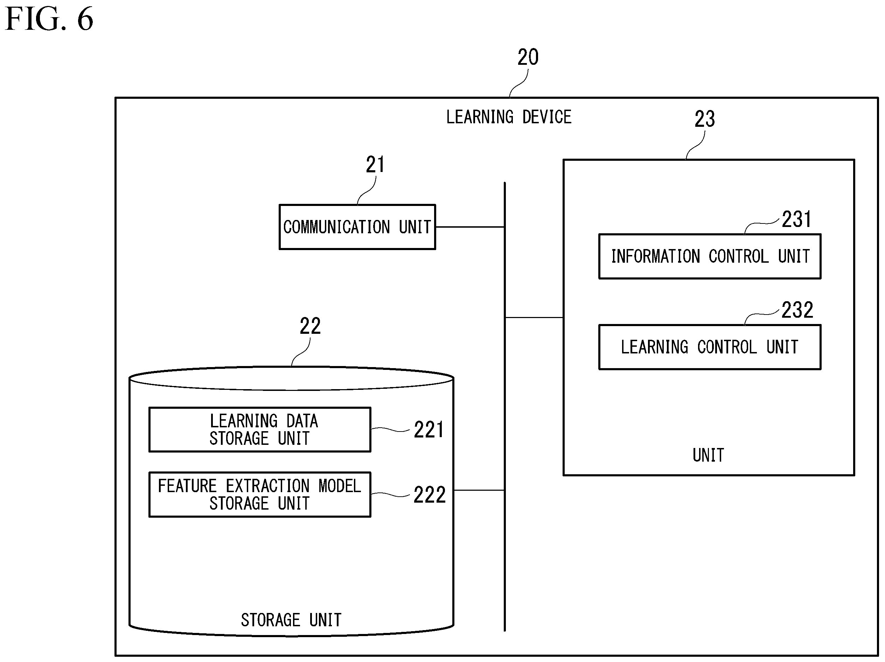

FIG. 6 is a schematic block diagram illustrating a specific example of a functional configuration of the learning device 20.

The learning device 20 is configured using an information processing device such as a personal computer or a server device, for example. The learning device 20 includes a communication unit 21, a storage unit 22, and a control unit 23.

The communication unit 21 is a communication device. The communication unit 21 may be configured as a network interface, for example. The communication unit 21 performs data communication with other devices via a network under control of the control unit 23. The communication unit 21 may be a device that performs wireless communication, or may be a device that performs wired communication.

The storage unit 22 is configured using a storage device such as a magnetic hard disk device or a semiconductor storage device. The storage unit 22 stores data used by the control unit 23. The storage unit 22 may function as, for example, a learning data storage unit 221 and a feature extraction model storage unit 222.

The learning data storage unit 221 stores learning data used in a learning process executed in the learning device 20. The learning data includes data of a plurality of multispectral images obtained by imaging a plurality of learning target animals. The learning target animals may be animals of the same type as or a different type from the target animal to be determined, as long as they are different individuals from the target animal to be determined. Further, a plurality of types of animals may be used as the animals to be learned, or one type of animal may be used. In this context, there is a possibility that the determination accuracy can be further improved when the learning target animals and the target animal to be determined are of more closely related types. The learning data further includes label information indicating a correct answer of information (concentration of a blood component) to be obtained as a determination result of a determination target captured in each multispectral image. A specific example of the blood component is glucose.

The feature extraction model storage unit 222 stores a trained model obtained by a learning process using the learning data stored in the learning data storage unit 221.

The control unit 23 is configured using a processor such as a CPU and a memory. The control unit 23 functions as an information control unit 231 and a learning control unit 232 by the processor executing a program. All or part of the functions of the control unit 23 may be implemented using hardware such as an ASIC, a PLD, or an FPGA. The above-mentioned program may be recorded on a computer-readable recording medium. The computer-readable recording medium is, for example, a portable medium such as a flexible disk, a magneto-optical disk, a ROM, a CD-ROM, or a semiconductor storage device (e.g., an SSD), or a storage device such as a hard disk or a semiconductor storage device built into a computer system. The above-mentioned program may be transmitted via an electric communication line.

The information control unit 231 controls input and output of information. For example, the information control unit 231 acquires learning data from another device (an information processing device or a recording medium) and records the acquired learning data in the learning data storage unit 221. For example, the information control unit 231 transmits the trained model stored in the feature extraction model storage unit 222 to another device (for example, the determination device 30).

The learning control unit 232 executes a learning process using learning data. As a specific example of such a learning process, for example, supervised learning for classification such as a support vector machine, a random forest, or a neural network may be used. For example, the learning control unit 232 performs supervised learning to thereby generate a trained model for outputting a concentration of a blood component based on an input multispectral image. The learning control unit 232 records the generated trained model in a feature extraction model storage unit 222. The trained model obtained by the learning control unit 232 may be transmitted to a determination device 30 and recorded in a trained model storage unit 321 of the determination device 30.

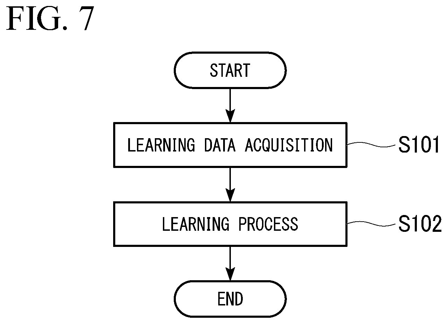

FIG. 7 is a flowchart showing a specific example of a process of the learning device 20.

First, the information control unit 231 acquires learning data (Step S101). The learning data may be, for example, input by a user, acquired from another information device via communication, or acquired from a recording medium connected to the learning device 20. The learning control unit 232 executes a learning process using the learning data, and records a trained model in the feature extraction model storage unit 222 (Step S102).

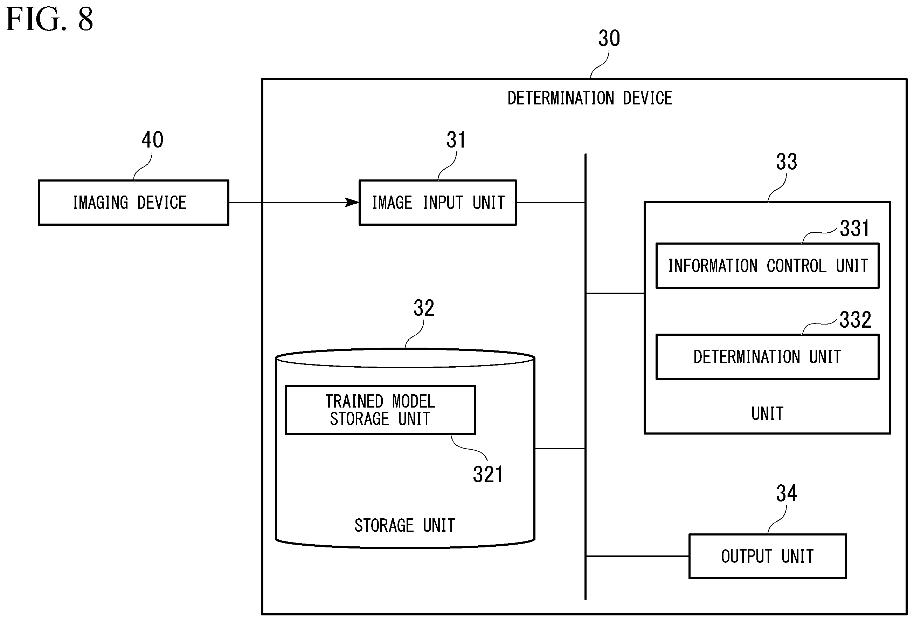

FIG. 8 is a schematic block diagram showing a specific example of a functional configuration of the determination device 30.

The determination device 30 is configured using an information processing device such as a smartphone, a tablet, a personal computer, or a dedicated device, for example. The determination device 30 includes an image input unit 31, a storage unit 32, a control unit 33, and an output unit 34.

The image input unit 31 receives data of a multispectral image input to the determination device 30. The image input unit 31 receives the input of the data of the multispectral image captured by the imaging device 40. A manner in which the image input unit 31 receives the input of the data of the multispectral image from the imaging device 40 is not limited. The image input unit 31 may be, for example, connected to the imaging device 40 via a USB (Universal Serial Bus) or another communication cable, and may receive the input of the data of the image via the communication cable. The image input unit 31 may be communicatively connected to the imaging device 40 via wireless communication such as Bluetooth (registered trademark), for example, and may receive the input of the data of the image.

The storage unit 32 is configured using a storage device such as a magnetic hard disk device or a semiconductor storage device. The storage unit 32 stores data used by the control unit 33. The storage unit 32 may function as, for example, a trained model storage unit 321.

The trained model storage unit 321 stores a trained model to be used when the determination unit 332 performs a determination process. The trained model is generated in advance by the learning device 20.

The control unit 33 is configured using a processor such as a CPU and a memory. The control unit 33 functions as an information control unit 331 and a determination unit 332 by the processor executing a program. All or a part of each function of the control unit 33 may be implemented using hardware such as an ASIC, a PLD, or an FPGA. The above-mentioned program may be recorded on a computer-readable recording medium. The computer-readable recording medium is, for example, a portable medium such as a flexible disk, a magneto-optical disk, a ROM, a CD-ROM, or a semiconductor storage device (e.g., an SSD), or a storage device such as a hard disk or a semiconductor storage device built into a computer system. The above-mentioned program may be transmitted via an electric communication line.

The information control unit 331 acquires a trained model from another device such as the learning device 20. The information control unit 331 acquires data of a multispectral image input from the image input unit 31. The information control unit 331 outputs information indicating a determination result obtained by the determination unit 332 to the outside via the output unit 34. For example, the information control unit 331 may transmit the information indicating the determination result to another device, or may output the information indicating the determination result from the output unit 34 via a display or a speaker.

The determination unit 332 performs a determination process using the trained model stored in the trained model storage unit 321 and the multispectral image input from the image input unit 31. A concentration of a blood component of an animal, which is a determination target and from which the multispectral image is captured, is determined by the determination process. Specifically, the process is as follows. The determination unit 332 acquires feature values relating to a plurality of values corresponding to wavelengths from image data of blood vessels of the animal generated by the imaging device 40. As described above, for example, the feature values are absorption spectra of blood components contained in blood in the blood vessels.

The output unit 34 outputs a signal to an output device such as a display or a speaker, for example. For example, the output unit 34 may output an image signal indicating a determination result or an audio signal indicating the determination result.

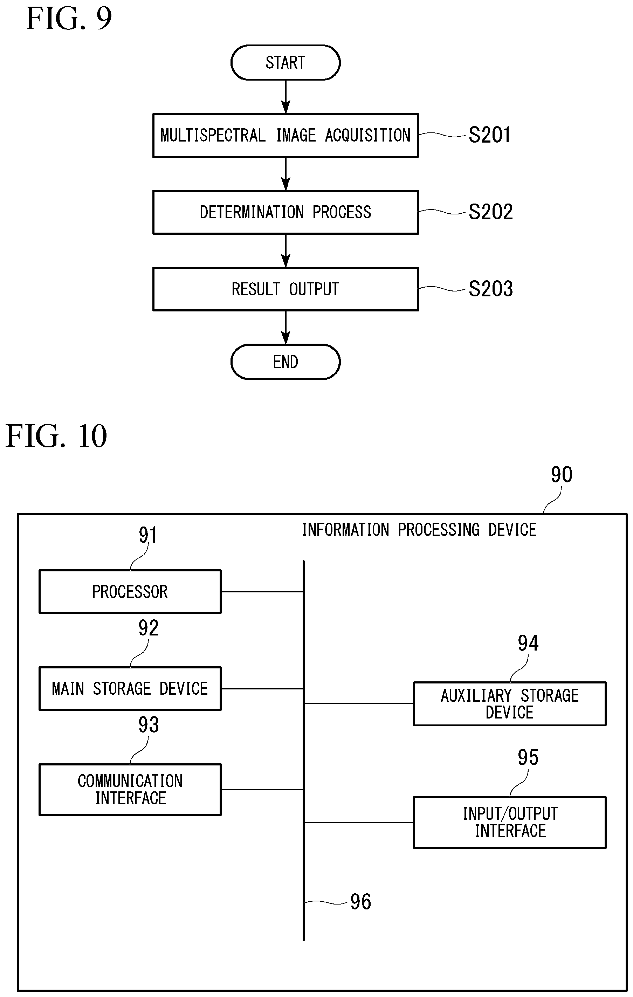

FIG. 9 is a flowchart showing a specific example of a process of the determination device 30.
First, the information control unit 331 acquires a multispectral image from the imaging device 40 (Step S201). The determination unit 332 performs a determination process using the multispectral image (Step S202). The determination unit 332 outputs information indicating the determination result from the output unit 34 to the output device (Step S203).

FIG. 10 is a diagram schematically showing an example of a hardware configuration of an information processing device 90 applied to the present embodiment.

The information processing device 90 includes a processor 91, a main storage device 92, a communication interface 93, an auxiliary storage device 94, an input/output interface 95, and an internal bus 96. The processor 91, the main storage device 92, the communication interface 93, the auxiliary storage device 94, and the input/output interface 95 are communicatively connected to each other via the internal bus 96. The information processing device 90 may be applied to, for example, the learning device 20 and the determination device 30. In such a case, for example, the communication unit 21 and the output unit 34 may be configured using the communication interface 93. For example, the image input unit 31 and the output unit 34 may be configured using the input/output interface 95. For example, the storage unit 22 and the storage unit 32 may be configured using the auxiliary storage device 94. Further, the control unit 23 and the control unit 33 may be configured using the processor 91 and the main storage device 92.

A display device 5 is a specific example of a device to which the output unit 34 outputs. The display device 5 displays a determination result obtained by the determination device 30. Examples of the display device 5 include a personal computer monitor, a liquid crystal display, an organic EL display, and a portable terminal such as a smartphone or a tablet personal computer. The display device 5 is connected to the determination device 30 via a wireless LAN (Local Area Network) or a wired LAN. If a portable terminal is used as the display device 5, an analyzer can confirm an estimation result at hand. Further, if the display device 5 is provided with an application for displaying the estimation result, the display device 5 can determine a health condition of a measuremnt target animal, according to the estimation result and can show a determination result indicating the health condition. The determination result of the health condition of the animal is displayed as, for example, "Excellent," "Good," "Poor," or the like. The determination device 30 and the display device 5 may be configured as an integrated device.

According to the determination system 10 of the present embodiment, since the imaging device 40 (for example, a multispectral camera) and the determination device 30 are provided, it is possible to non-invasively grasp the health condition of the target animal on the spot.

### EXAMPLES

Hereinbelow, the present invention will be described with reference to Examples which, however, should not be construed as limiting the present invention.

### [Example 1]

The surface of the base of a cattle's tail was imaged with a multispectral camera to generate image data of the cattle's blood vessels. As the multispectral camera, a near-infrared spectrometer "S-G1" manufactured by nanoseed Inc. was used.

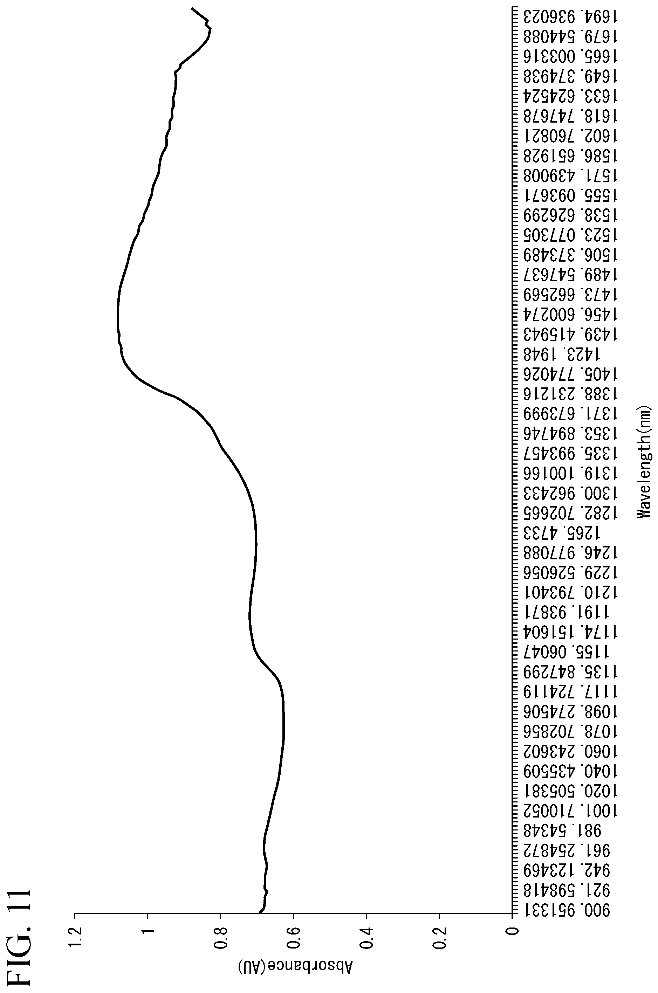

Next, from the image data of the cattle's blood vessels generated by the multispectral camera, 46 absorbances relating to the inside of the cattle's blood corresponding to wavelengths in a range of 900 nm or more and 1694 nm or less were acquired, and an absorption spectrum shown in FIG. 11 was obtained.

Based on the obtained absorption spectrum, a concentration of glucose contained in the cattle's blood was estimated.

Table 1 shows an example in which the range of measured values of blood glucose concentration and the number of samples in the case of blood sampling are classified. Table 2 shows the classification accuracy of the concentration of glucose contained in the cattle's blood estimated by the analysis method of the present invention for each class.

**[Table 1]**

| Class | 0 | 1 | 2 | 3 | 4 | 5 |
|---|---|---|---|---|---|---|
| Range [mg/dl] | 57 to 59 | 60 to 64 | 65 to 67 | 68 to 70 | 71 to 75 | 76 to 83 |
| Samples | 18 | 33 | 23 | 23 | 29 | 14 |

**[Table 2]**

| Class | 0 | 1 | 2 | 3 | 4 | 5 | Average |
|---|---|---|---|---|---|---|---|
| Accuracy [%] | 94.44 | 98.75 | 99.57 | 97.39 | 93.10 | 100 | 97.05 |

From the results shown in Tables 1 and 2, the classification accuracy of the glucose concentration was 97. 05%. Therefore, it was confirmed that the analysis method of the present invention is an effective method for estimating the concentration of a component such as glucose contained in the blood of an animal.

### [Example 2]

The surface of the base of a cattle's tail was imaged with a multispectral camera to generate image data of the cattle's blood vessels. As the multispectral camera, a near-infrared spectrometer "S-G1" manufactured by nanoseed Inc. was used.

Next, from the image data of the cattle's blood vessels generated by the multispectral camera, 46 absorbances relating to the inside of the cattle's blood corresponding to wavelengths in a range of 900 nm or more and 1694 nm or less were acquired, and an absorption spectrum was obtained.

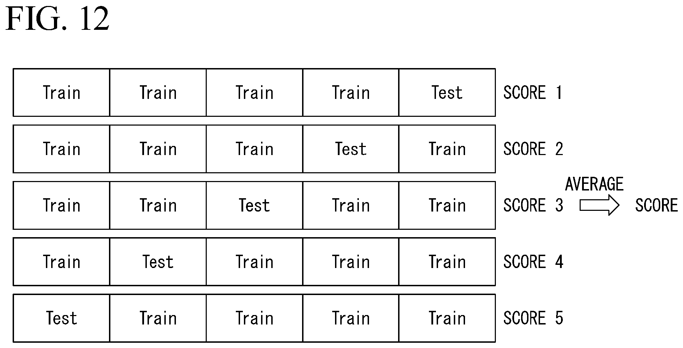

As shown in FIG. 12, the data of the obtained absorption spectrum was divided into four pieces of training data (Train) and one piece of test data (Test), and learning and verification of a classification model were performed.

The learning and verification were repeated five times while changing the training data and the test data, and an average value of scores was used as an evaluation value of the classification model.

Absorption spectra were measured a total of 303 times over eight months for 22 Japanese Black cattle.

The data was divided into 242 measurements for training data and 61 measurements for test data such that the ratio of the number of data for each label was the same, and the learning and verification of the classification model were performed.

The results are shown in Table 3.

**[Table 3]**

| Label No. | 0 | 1 | 2 | 3 | 4 | 5 |
|---|---|---|---|---|---|---|
| Concentration range | 57 to 61 | 62 to 65 | 66 to 69 | 70 to 73 | 74 to 78 | 79 to 83 |
| [mg/dl] | | | | | | |
| Number of data | 81 | 82 | 51 | 65 | 15 | 9 |

### EXPLANATION OF REFERENCE NUMERALS

- 10: Determination system
- 20: Learning device
- 30: Determination device
- 40: Imaging device
- 5: Display device

## Claims

1. An analysis method comprising:
a first step of obtaining feature values relating to a plurality of values corresponding to wavelengths from image data of a blood vessel of a target animal, the image data being generated by capturing an image of a shallow part of a body surface (vein) of the animal with a multispectral camera; and
a second step of estimating a concentration of a blood component contained in blood in the blood vessel based on the feature values.

2. The analysis method according to claim 1, wherein the plurality of values corresponding to wavelengths are values relating to absorbance of the blood in the blood vessel.

3. The analysis method according to claim 1, wherein:
in the first step, at least n (where n ≥ 228) values are obtained at wavelengths of 900 nm or more and 1,694 nm or less; and
in the second step, the concentration of the blood component is classified into classes according to the concentration of the blood component, based on feature values relating to a plurality of values including the n values.

4. The analysis method according to claim 1, wherein the image data of the blood vessel is image data of a ventral side of a tail (median caudal vein) of the target animal.

5. A determination system comprising:
an imaging device that captures an image of a body surface of a target animal;
a learning device that obtains feature values relating to a plurality of values corresponding to wavelengths from image data of a blood vessel of the animal generated by the imaging device, and learns the feature values; and
a determination device that estimates a concentration of a blood component contained in blood in the blood vessel based on the feature values.
